# EUROPEAN PATENT SPECIFICATION

(11) **EP 0 805 203 B1**
(45) Date of publication and mention of the grant of the patent: **01.08.2007**
(21) Application number: 97201105.0
(22) Date of filing: 15.09.1993
(51) Int. Cl.: C12N 15/12, C07K 16/28, A61K 38/18, A61K 39/395

(54) **Medical use of an antibody or antibody fragment against the extracellular domain of Met for the prevention of metastasis**
Medizinische Verwendung eines Antikörpers oder eines Antikörperfragments gegen die extrazelluläre Domäne von Met zur Prevention von Metastasen
Utilisation medicale d'un anticorps or d'un fragment d'un anticorps dirigé contre le domaine extracellulaire de Met pour la prévention de métastases.

(30) Priority: 18.09.1992 US 946061
(43) Date of publication of application: 05.11.1997
(62) Divisional of application: 93921471.4
(73) Proprietor: THE GOVERNMENT OF THE UNITED STATES OF AMERICA as represented by THE SECRETARY OF THE DEPARTMENT OF HEALTH AND HUMAN SERVICES, Rockville, MD 20892-9902 (US)
(72) Inventor: Faletto, Donna L., Mount Airy, MD 21771 (US); Tsarfaty, Ilan, Ramat-Aviv, Tel Aviv 69978 (IL); Rong, Sing, Frederick, MD 21702 (US); Oskarsson, Marianne, Gaithersburg, MD 20879 (US); Vande Woude, George F., Berryville, VA 22611 (US)
(74) Representative: Gardner, Rebecca Katherine

(56) References cited:
- EP-A- 0 456 188
- WO-A-91/12272
- WO-A-92/05184
- WO-A-92/13097
- WO-A-93/15754
- WO-A-93/23541
- EMBO J., vol. 11, no. 7, July 1992, OXFORD UNIVERSITY PRESS,GB;, pages 2503-2510, XP002036379 N.A. LOKKER ET AL.: "structure-function analysis of hepatocyte growth factor: identification of variants that lack mitogenic activity yet retain high affinity receptor binding"
- BIOCHEMICAL AND BIOPHYSICAL RESEARCH COMMUNICATIONS, vol. 180, no. 2, 31 October 1991, pages 1151-1158, XP000230641 NOBUYUKI SHIMA ET AL: "TUMOR CYTOTOXIC FACTOR / HEPATOCYTE GROWTH FACTOR FROM HUMAN FIBROBLASTS: CLONING OF ITS CDNA, PURIFICATION AND CHARACTERIZATION OF RECOMBINANT PROTEIN"
- WEIDNER ET AL.: "Evidence for the identity of human scatter factor and human hepatocyte growth factor." PROC. NATL.ACAD.SCI, vol. 88, August 1991 (1991-08), pages 7001-7005, USA

## Description

### Background of the Invention

Hepatocyte growth factor (HGF) was first purified from human and rabbit plasma and rat platelets on the basis of its ability to stimulate mitogenesis of rat hepatocytes. (E. Gohda et al., J. Clin. Invest 81: 414 (1988); R. Zarnegar and G. Michalopoulos, Cancer Res. 49: 3314 (1989); T. Nakamura et al. FEBS Lett. 224: 311 (1987)). Thus, HGF may act as a humoral factor promoting liver regeneration after partial hepatectomy or liver injury. (E.H. Gohda et al., J. Clin. Invest. 81: 414-419 (1988); G.K. Michalopoulos, FASEB J. 4: 176 (1990)). The same factor was purified from human fibroblast culture medium and shown to act on melanocytes and a variety of epithelial and endothelial cells. (T. Igawa et al., BBRC 174: 831-838 (1991); M. Kan et al., BBRC 174: 331-337 (1991) and J.S. Rubin et al., Proc. Nat'l. Acad. Sci. U.S.A. 88: 415 (1990)). Together with evidence of HGF expression in several organs (J.S. Rubin et al., Proc. Nat'l. Acad. Sci. U.S.A. 88: 415 (1990); K. Tashiro et al. Proc. Nat'l. Acad. Sci. U.S.A. 87: 3200 (1990); R. Zarnegar et al., Proc. Nat'l. Acad. Sci. U.S.A. 87: 1252 (1990); T. Kinoshita et al., Biochem. Biophys. Res. Comm. 165: 1229 (1989)), these findings indicate that HGF may also act as a paracrine mediator of proliferation for a broad spectrum of cell types. Molecular cloning of HGF revealed a remarkable structural homology to plasminogen and related serine proteases (J.S. Rubin et al., Proc. Nat'l. Acad. Sci. U.S.A. 88: 415 (1990); T. Nakamura et al., Nature 342: 440 (1989); K. Miyazawa et al., Biophys. Res. Comm. 163: 967 (1989)). Recent evidence that HGF induces rapid tyrosine phosphorylation of proteins in intact target cells suggests that a tyrosine kinase receptor mediates its mitogenic signal (J.S. Rubin et al., Proc. Nat'l. Acad. Sci. U.S.A. 88: 415 (1990)).

HGF is structurally related to the family of serine proteases that includes plasminogen, prothrombin, urokinase, and tissue plasminogen activator (J.S. Rubin et al., Proc. Nat'l. Acad. Sci. U.S.A. 88: 415 (1990); T. Nakamura et al., Nature 342: 440 (1989)). For instance, HGF structurally resembles plasminogen in that it possesses characteristic kringle domains (Patthy et al. FEBS Lett. 171: 131-136 (1984)) and a serine protease domain (Miyazawa et al., Biochem. Biophys. Res. Commun. 163: 967-73 (1989); Nakamura et al., Nature 342: 440-43 (1989)). As defined in the present invention, HGF includes a growth factor previously characterized as a broad-spectrum mitogen called plasminogen-like growth factor (PLGF), the subject matter of U.S. application serial no. 07/582,063. Several proteases, including members of the serine protease family, stimulate DNA synthesis presumably through a proteolytic mechanism similar to tryptic activation of the insulin receptor. (S.E. Shoelson et al. J. Biol. Chem. 263: 4852 (1988)). Only urokinase has been found to associate with a specific cell-surface receptor, which itself bears no homology to any known tyrosine kinase receptors (A.L. Roldan et al., EMBO J. 9: 467 (1990)).

Scatter factor (SF) originally had been considered to be related to but different from HGF, SF being associated with cell motogenicity (motility), and HGF being associated with cell mitogenicity (growth). However, recent work has demonstrated that SF and HGF are, in fact, the same protein having the identical amino acid sequence, the same receptor which is the protein encoded by the *c-met* proto-oncogene, and same biochemical affect (E. Gherardi and M. Stoker, Nature 346: 228 (1990); K.M. Weidner et al., PNAS 88: 7001-7005 (1991); M. Bhargava, et al., Cell Growth & Diffn. 3(1): 11-20 (1992); Naldini et al., EMBO J. 10(10): 2867-78 (1991); E. Gherardi and M. Stoke, Cancer Cells 3:(6): 227-32 (1991) and Graziani et al., J. Biol. Chem. (USA) 266

The subject matter of U.S. Serial No. 07/642,971, incorporated by reference above, describes a complex comprising HGF/SF and *met* protooncogene protein ("Met"), and identifies Met as the receptor for HGF/SF. The *met* protooncogene protein is a member of the tyrosine kinase growth factor receptor family. Knowledge of this receptor/ligand relationship facilitates the study of proliferative disorders and tumorigenicity in which expression of these molecules may play an important role. Additionally, identification of the *met* protooncogene receptor HGF/SF complex provides a means for identifying tissues other than liver tissue affected by factor binding.

Evidence suggests that the positive effects of growth factors on cell proliferation can be counteracted at a variety of levels, both intracellularly (Moses et al. Cell 63: 245-247 (1990)) and at the cell surface (Hannum et al., Nature 343: 336-340 (1990); Eisenberg, et al., Nature 343: 341-346 (1990); Carter et al., Nature 344:633-637 (1990)).

Various sources of human HGF have been identified (Nakamura, T., Progress in Growth Factor Research, 3: 67-86 (1992)) and it has been shown that the gene product can be overexpressed when transfected into *Cos* cells or by baculovirus host systems. (Nakamura et al., Nature 342:440-43 (1989); Cooper, et al., The EMBO J., 5(10): 2623-2628 (1986)). A mammalian cell line continuously shedding large amounts of human HGF/SF has yet to be identified.

### Summary of the Invention

Accordingly, the present invention involves the use of a substance which prevents hepatocyte growth factor/scatter factor (HGF/SF) from binding with *met* protooncogene protein (Met), wherein said substance is an antibody or antibody fragment against the extracellular domain of Met, in the preparation of a medicament for use in a method of preventing tumor cell metastasis, wherein in said method, a tumor-bearing mammal is treated with an effective inhibiting amount of said substance.

Binding may be inhibited by a substance which is an HGF/SF variant, mimetic, or antibody or antibody fragment against HGF/SF, which prevents HGF/SF from binding with Met. Binding may also be inhibited by a Met variant, Met mimetic and antibody or antibody fragment against Met, which similarly prevents HGF/SF-Met binding.

Furthermore, in view of need for a continuously producing source of large quantities of HGF/SF, applicants have discovered and describe herein a mouse NIH/3T3 cell line which can express unexpectedly high levels of HGF/SF by recombinant methods.

A method of producing HGF/SF comprises the steps of:
(a) transfecting NIH/3T3 cells with DNA encoding HGF/SF^{hu} and Met^{hu};
(b) introducing cells transfected in accordance with step (a) into a mammal, thereby generating a primary tumor;
(c) explanting and propagating cells of the primary tumor *in vitro*;
(d) selecting those cells propagated in accordance with step (c) which express high levels of HGF/SF^{hu} and high levels of Met^{hu};
(e) introducing cells selected in accordance with step (d) into a mammal, thereby producing a secondary tumor;
(f) explanting and propagating cells of the secondary tumor *in vitro;* and
(g) obtaining HGF/SF^{hu} produced by the cells of step (f) .

A cell line co-transfected with a first DNA vector comprises, in operable linkage, a promoter derived from a long terminal repeat of a retrovirus, DNA encoding the entire coding domain of human Met and a polyadenylation signal and a second DNA vector comprising, in operable linkage, a promoter derived from a long terminal repeat of a retrovirus, DNA encoding human HGF/SF and a polyadenylation signal.

### Brief Description of the Drawings

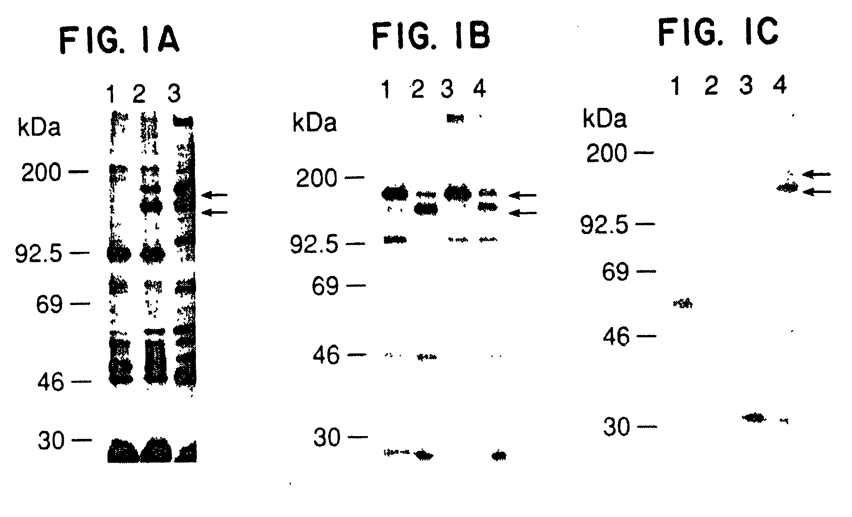

Figure 1 demonstrates *met* products in transfected NIH/3T3 cells. (A) **Immunoprecipitation analysis**. Cells were metabolically labeled with [³⁵S]methionine and [³⁵S]cysteine (Translabel, ICN) for 5 hours in DMEM lacking methionine and cysteine (Gibco) and cell lysates were immunoprecipitated with either a --------FAX--------Met^{hu}-specific monoclonal antibody, 19S (Faletto, D.L. et al., Oncogene 7(6): 1149-1157 (June 1992) (lanes 1 and 2), or Met^{mu}-specific peptide antibody, SP260 (Iyer, A. et al., Cell Growth & Diff. 1: 87-95 (1990)) (lane 3). Immunoprecipitation was completed with protein G-sepharose (Gibco), the complex was solubilized in SDS sample buffer with 5% β-mercaptoethanol, and resolved on 7.5% acrylamide gels. In lane 1, cells were transfected with pSV2neo only; in lane 2, cells were transfected with *met^{hu};* in lane 3, cells were transfected with *met*^{mu}. (B) **Pulse-chase analysis**. Cells were metabolically labeled with [³⁵S]methionine and [³⁵S]cysteine for 45 minutes (lanes 1 and 3), followed by a chase period of 3 hours (lanes 2 and 4). Met was immunoprecipitated with 19S monoclonal antibody against Met^{hu} (lanes 1 and 2) or SP260 peptide antibody, against Met^{mu} (lanes 3 and 4) and subjected to electrophoresis as in panel A. In lanes 1 and 2 are cells transfected with *Met*^{hu}; in lanes 3 and 4 are cells transfected with *Met*^{mu}. (C) **Cell surface iodination of *met***. Near-confluent cells were pelleted and labeled with Na¹²⁵I in the presence of Iodo-Gen (Pierce). The labeled cells were washed three times with PBS, lysed with RIPA buffer, and subjected to immunoprecipitation with either 19S monoclonal antibody (lanes 1 and 2) or SP260 peptide antibody (lanes 3 and 4). Met^{hu} expressing NIH/3T3 cells are in lanes 1 and 3. Met^{mu} expressing NIH/3T3 cells are in lanes 2 and 4. Arrows indicate the positions of p170^{met} and p140^{met}.

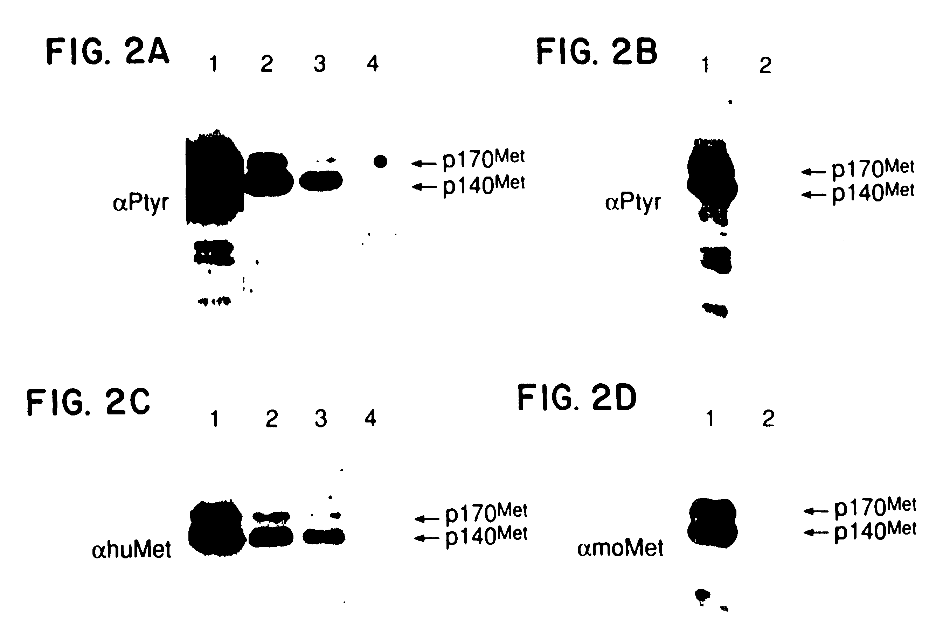

Figure 2 shows *met* product reactivity with anti-P-Tyr antibody. Near-confluent cells on a 100-mm dish were washed twice with cold TBS and lysed in 1 ml of lysis buffer (25 mM Tris-HCl, pH 8.0, 100 mM NaCl, 50 mM NaF, 1% Triton X-100, 10 µg/ml aprotinin, 10 µg/ml leupeptin, 1.25 mM PMSF, 1 mM sodium orthovanadate). One milligram of protein was immunoprecipitated with anti-C28 peptide antibody for Met^{hu}, (Gonzatti-Haces, M. et al. Proc. Nat'l. Acad. Sci. U.S.A. 85: 21-25 (1988)) (panels A and C) or peptide antibody SP260 for Met^{mu} (Iyer, A., et al., Cell Growth & Diff. 1: 87-95 (1990)) (panels B and D). After dissolving in SDS buffer, samples were separated by SDS-PAGE on 7.5% gels, transferred to Immobilon-P (Millipore), and probed with anti-P-Tyr antibody, 4G10, Morrison, D.K., et al., Cell 58: 649-657 (1989) (panels A and B), 19S monoclonal antibody (panel C), or mouse *met* peptide antibody, SP260 (panel D). The Immobilon filter was then incubated with ¹²⁵I-protein A (ICN) and subjected to autoradiography. Lanes 1-3 of panels A and C have three different lines of *met*^{hu}-transfected cells; lane 4 has NIH/3T3 control cells. Lane 1 of panels B and D are cells transfected with *met*^{mu}. Lane 2 has NIH/3T3 control cells.

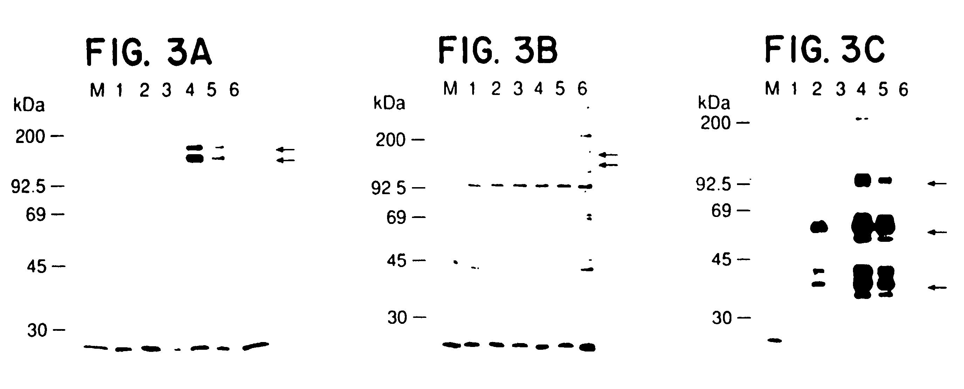

Figure 3 shows the characterization of Met^{hu} and HGF/SF^{hu} in NIH/3T3 tumor cells. Tumor cells were explanted and metabolically labeled with [³⁵S]methionine and [³⁵S]cysteine for 6 hours. Cell lysates were immunoprecipitated with either 195 monoclonal antibody (panel A), or peptide antibody SP260 (panel B). Then, 0.25 ml of the "6 hour" supernatants was concentrated threefold in Centricon (Amicon; 10K cut-off); the volumes were adjusted to 0.3 ml with RIPA buffer and the samples were immunoprecipitated with anti-HGF monoclonal antibody A3.1.2 (panel C). Lanes 1 and 3 are samples from two different lines of cotransfected cells before injection. Lane 2 is a tumor explant derived from the cells analyzed in lane 1; lanes 4 and 5 are tumor explants derived from the cells analyzed in lane 3. Lane 6 is a sample prepared from control NIH/3T3 cells. Arrows indicate the positions of p170^{met} and p140^{met} (panels A and B) and the positions of the 87 kDa (the precursor), 69 kDa, and 34 kDa HGF polypeptides (panel C).

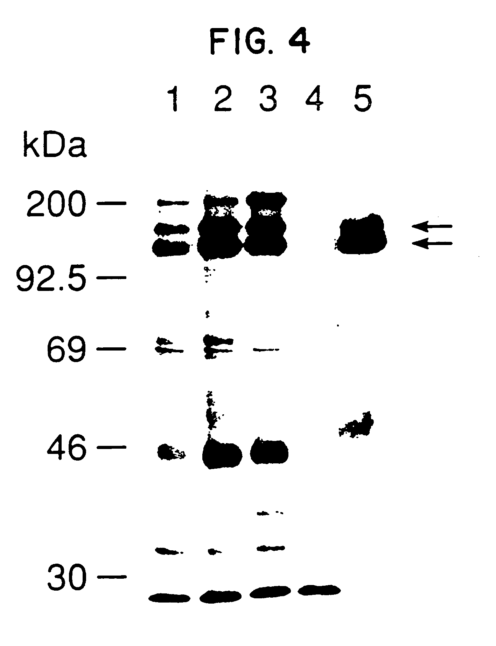

Figure 4 demonstrates the characterization of Met chimeric protein in NIH/3T3 cell tumors. Cells were metabolically labeled with [³⁵S]methionine and [³⁵S]cysteine for 6 hours and cell lysates were immunoprecipitated with 19S monoclonal antibody (lanes 1-4). In lane 1 are uninjected G418 (Gibco)-resistant cells transfected with mouse N-terminal/human C-terminal chimeric *met*; in lanes 2 and 3 are tumors derived from cells analyzed in lane 1; in lane 4, NIH/3T3 control cells; in lane 5, Western blot of the same cells as in lane 1, analyzed with anti-P-Tyr antibody (4G10) following immunoprecipitation with human anti-C28 peptide antibody.

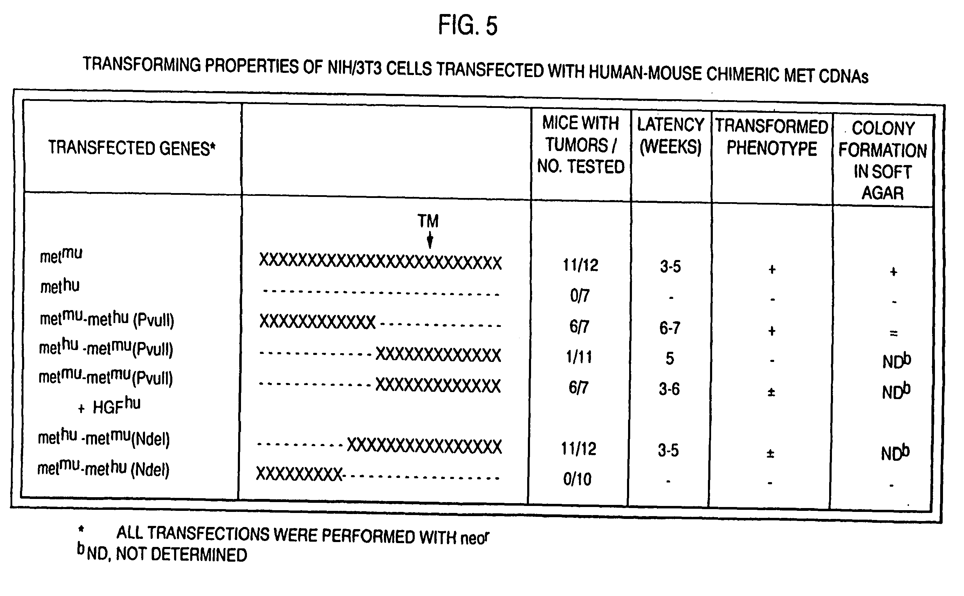

Figure 5 shows that the extracellular domain of Met^{mu} confers transforming potential onto Met^{hu} (line 3 compared to line 2) and the Met^{hu} extracellular domain is only transforming when co-transfected with HGF^{hu} (compare lines 4 to 5). Furthermore, the last two lines delineate the region of *met*^{mu} between the NdI-Pvu II sites as a region conferring transforming potential onto Met^{hu} (compare line 6 to line 4).

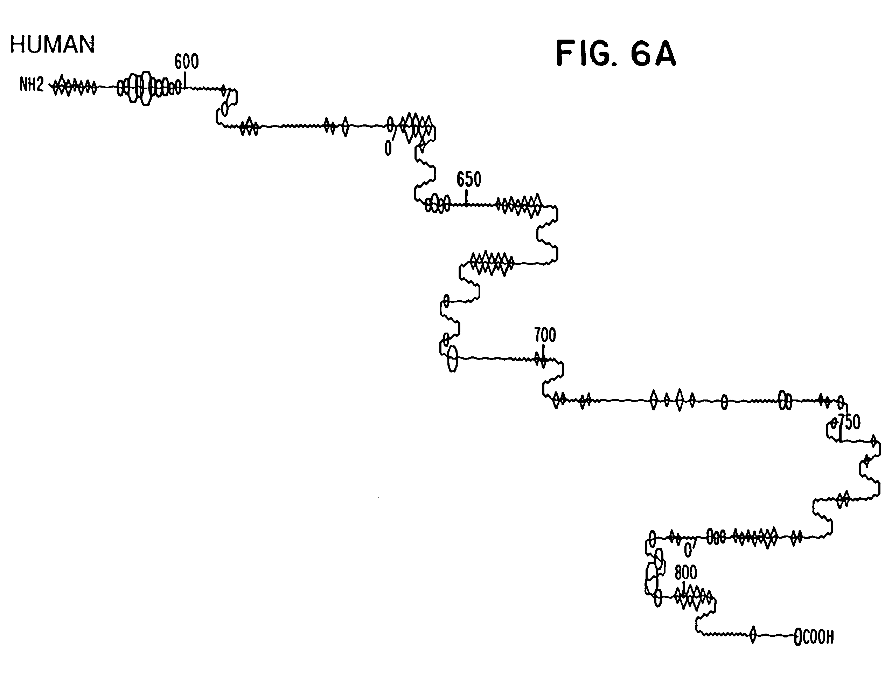

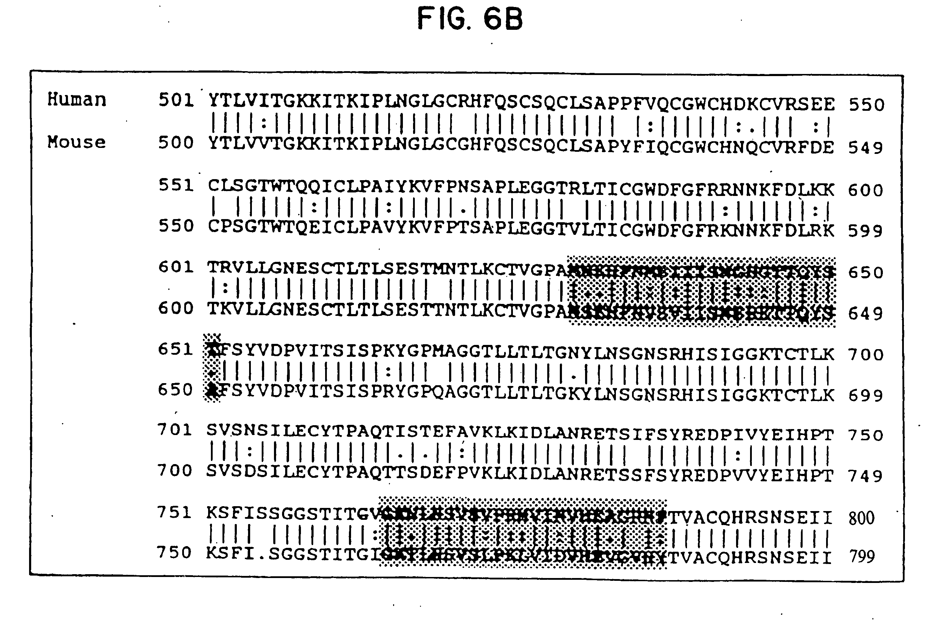

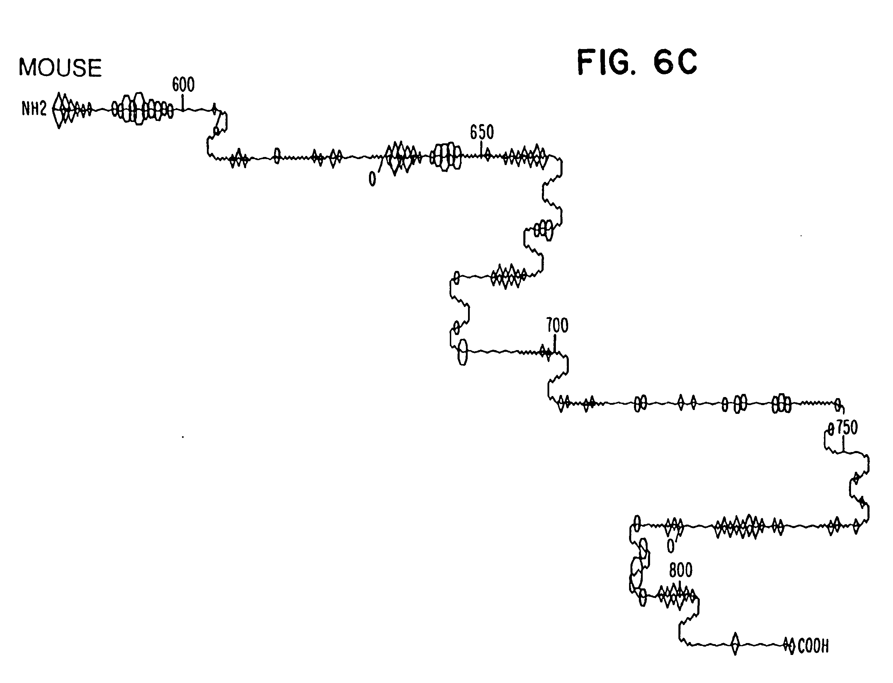

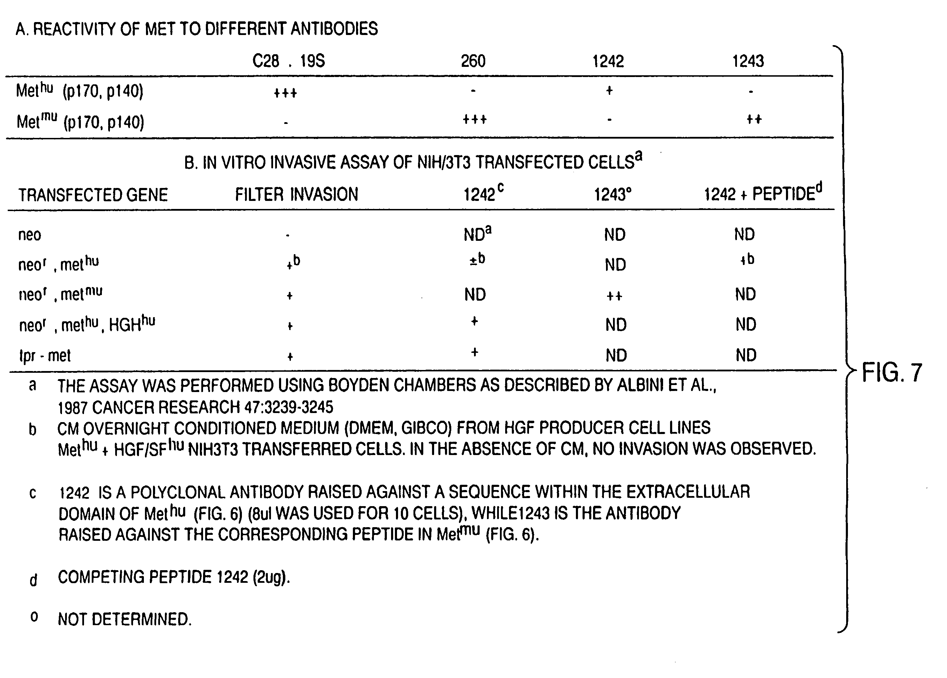

Figure 6 shows a comparison of the computer-generated predicted structure of the amino acid sequence between the conserved Nde I - Pvu II sites in the human Met (top) with mouse Met sequence (bottom). The amino acid sequence of human and mouse Met (between the Nde I - Pvu II sites) is depicted in the inset box - highlighted are regions where the amino acid sequence is less conserved (conservation is depicted by dashed lines between the human and mouse sequence). The less conserved domains within the Nde I - Pvu II segment of cDNA conferring transforming potential onto Met either reflect domains directly involved in ligand binding, or through structural characteristics modulate either ligand binding or activation of the receptor following ligand binding. Therefore, applicants generated polyclonal antibodies to synthetic peptides corresponding to these sequences. Antibodies to the first domain were generated in rabbits against human sequence (α 1240) and mouse sequence (α 1241); antibodies to the second domain were generated against human sequence (α 1242) and mouse sequence (α 1243). Only 1242 and 1243 precipitated human and murine *c-met* protein, respectively.
Figure 7 depicts the reactivity of human versus mouse Met to the human (C28, 19S, 1242) and mouse (260, 1243) specific antibodies (part A). Part B demonstrates that when mouse *met* cDNA is transfected into NIH/3T3 cells (which express HGF/SF^{mu}, endogenously) the cells are invasive in a Boyden chamber assay. (Albini et al. Cancer Res. 47: 3239-3245 (1987)). That is, the cells move across a filter coated with matrigel (a basement membrane-like compound). Cells transfected with the oncogenic form of *met* (tpr-*met*) are also highly invasive in this assay. For cells to invade across the filter, they must not only be motile, but must be able to degrade basement membrane components (and thus secrete enzymes such as collagenase or plasminogenase). *Met*^{hu} transfectants (line 2) are not invasive in the absence of human ligand, HGF/SF^{hu}, but are invasive when conditioned media from the HGF producer cell line is added. On the other hand, contransfectants of *met^{hu}* and HGF/SF^{hu} (line 5) are invasive in this assay

The second column on this figure shows that 1242 antibody to human Met within the domain described above inhibits filter invasion of the *met*^{hu} transfectants, co-cultured with conditioned media containing HGF/SF^{hu}. The last column shows that this inhibition is blocked in the presence of competing peptide. On the other hand, the invasive capacity of cotransfectants producing Met^{hu} and HGF^{hu} (line 4) is not blocked by the 1242 antibody, suggesting internal, intracellular autocrine activation of receptor by co-produced HGF/SF ligand.

Lastly, this figure shows that 1243 antibody did not block invasion of the filter by *met*^{mu} transfectants (line 3, column 3).

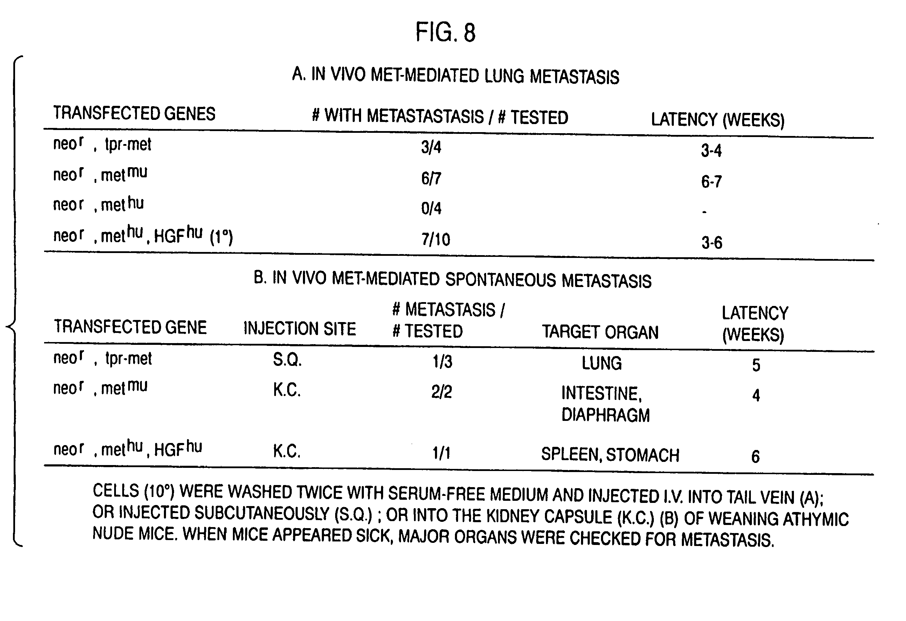

Figure 8 shows *in vivo* metastasis data showing that just as *met*^{mu} and tpr-*met* transfectants are tumorigenic *in vivo* (Figure 5), these cells are also metastatic in several types of assays (panels A and B). Furthermore, Met^{hu} (panel A) is not metastatic *in vivo* unless cotransfected with HGF^{hu} cDNA.

Figure 9 is a diagram depicting the construction of plasmids pRS2 and pRS24 which are co-transfected into NIH/3T3 cells in the production of HGF/SF, as described in Example 2.

### Detailed Description of the Preferred Embodiments

The present invention involves the use of a substance which prevents hepatocyte growth factor/scatter factor (HGF/SF) from binding with *met* protooncogene protein (Met), wherein said substance is an antibody or antibody fragment against the extracellular domain of Met, in the preparation of a medicament for use in a method of preventing tumor cell metastasis, wherein in said method, a tumor-bearing mammal is treated with an effective inhibiting amount of said substance. By the term "preventing" is meant diminishing or arresting tumor metastasis.

Preferably, the antibody is a polyclonal antibody. In a preferred embodiment, the antibody is against the sequence GKNLNSVSVPRMVINVHEAGRNF.

It is generally accepted in the art that for tumor cells to become "metastatic", a number of coordinated events must take place. For instance, metastasis involves the process whereby cancer cells penetrate the walls of the vascular and lymphatic circulatory systems, enter the circulatory system, lodge ("adhere") in a "downstream" capillary bed or lymph node and leave ("movement/mobility") the circulatory system to penetrate into normal stromal tissue. For these processes to occur, the tumor cell must acquire invasive properties. Invasive properties include (1) the ability of cells to adhere to the extracellular matrix (due to receptors on cell surface - such as the integrins); (2) the induction of destruction enzymes ("metalloproteases") which break through the extracellular matrix - such as collagenases and plasminogenases; and (3) the migration of cells through the "holes" created by enzymatic destruction. Increases in the levels of metalloproteases secreted by cells, increases in motogenicity of cells, and changes in the pattern of expression of receptors, involved in attachment (integrins) have all been noted, and in some cases, correlate with the metastatic potential of human carcinoma cells. (L.A. Liotta, Scientific American, 54-63 (Feb. 1992)).

It is known that HGF/SF increases the levels of activity of at least two of these events: the secretion of collagenases and plasminogenases and motogenicity. Furthermore, HGF/SF is known to be angiogenic and angiogenesis is important for the survival of tumor cells. (J. Behrens et al., J. Cell Biol. 108: 2435-2447 (1989); K.M. Weidner et al., J. Cell Biol. 111: 2097-2108 (1990)). By the term "motogenesis" is meant the process whereby continuous sheets of cells disaggregate, change morphology and become motile. (E. Gherardi and M. Stoker, Cancer Cells 3: 227-232 (1991)). This phenomenon is also referred to as "scattering."

Applicants have demonstrated that HGF/SF induces motogenicity in two ways. *See* Example 1, below. First, as shown in Table 1, cells which display a classical scattering response to HGF/SF showed rapid phosphorylation of endogenous *met* receptor on tyrosine, suggesting that scattering is activated through the *met* receptor.

**Table 1.**

| cell Line | Mitogenicity Index | Scattering (w/HGF) |
|---|---|---|
| Bix2H'HA | 1 | *** |
| Calu-1 | 1 ± | |
| SW620 | 1.7 | ++ |
| HT29 | 1 | + |
| HCT116 | 1.5 | + |

| | | |
|---|---|---|
| ¹Mitogenicity index = ³H-thymidine incorporation in presence of HGF/³H-thymidine incorporation without HGF. | | |

Secondly, a vigorous testing was performed on mouse breast tumor "epithelial" cell line (C127 cells) transfected with mouse and human *met* cDNA's encoding the *met* proto-oncogene product, to show scattering in response to HGF/SF only in cells that express the *met* product from exogenously introduced *met* cDNA, but not in the non-transfected cells. These results are shown in Table 2:

**Table 2.**

| Cell Line | Mitogenicity Index | Scattering (w/HGF) |
|---|---|---|
| C127 | 3^{a} | -- |
| C127 - *met*^{hu} | 3 | ++ |
| C127 - *met*^{mu} | 3 | + |

| | | |
|---|---|---|
| ^{a}Stimulation of ³H-thymidine incorporation is presumed to be due to low levels of exogenous *c-met* product in C127 cells. | | |

Inactivation of the HGF/SF-Met pathway provides the basis for the therapeutic methodologies of the present invention, designed to prevent tumor cell metastasis. These methodologies include the production of substances which prevent the binding of HGF/SF with Met. Such substances include, but are not limited to, HGF/SF or Met variants, HGF/SF or Met mimetics, and antibodies or antibody fragments against HGF/SF or Met which prevent HGF/SF binding with Met.

"Variants" include, for example, oligopeptides and polypeptides which are HGF/SF species that lack or possess impaired *met*-binding domain, or that lack or possess an impaired activating domain, but that otherwise retain the structural and biochemical characteristics of HGF/SF. Similarly, variants include Met species that lack or possess an impaired HGF/SF-binding domain, or lack or possess an impaired tyrosine kinase domain, but which otherwise retain the structural and biochemical characteristics of the *met* protein. *See, e.g.,* Lokker et al., EMBO J. 11(7): 2503-2510 (1992). HGF/SF and Met species which qualify as variants can be produced by conventional genetic engineering techniques. For example, variants can be produced by techniques which involve site-directed mutagenesis. *See* "Mutagenesis of Cloned DNA," in Current Protocols in Molecular Biology, 8.0.3 et seq. (Ausubel, et al. eds. 1989); Lokker *et al., supra.*

Variants also include, for instance, a soluble form of Met consisting of the extracellular HGF/SF-binding domain that acts as an antagonist of normal Met binding with HGF/SF. Such variants can be produced through molecular modelling techniques well known in the art of the invention. *See*, *e.g.,* Fuh et al., Science 256: 1677-1680 (1992).

A metastasis-inhibiting variant also can be a naturally occuring variant, such as HGF/NK2 or HGF/NK1, disclosed in U.S. co-pending application serial number 07/655,502, which is hereby incorporated by reference. HG/NK2 is a truncated form of HGF/SF encoded by alternative HGF transcripts which specify a sequence that includes the N-terminal and first two kringle domains. HGF/NK1 is another truncated form of HGF/SF encoded by HGF/SF transcripts which specify the sequence that includes the N-terminal and only the first kringle domain.

The metastasis-inhibiting substance may be an HGF/SF or Met "mimetic". One Example of a mimetic is an anti-idiotype antibody, that is, an antibody which is produced by immunizing an animal with an antibody which specifically binds to an epitope on an antigen. The anti-idiotype antibody recognizes and conforms to the combining site on the first antibody. Therefore, the shape of its combining site closely resembles the epitope which fits into the combining site of the first antibody. Because an anti-idiotype antibody has a combining site which mimics the original antigen, it can be used as a ligand for binding with the receptor to the original antigen. *See* Fineberg & Ertl, CRC Critical Reviews in Immunology 7: 269-284 (1987). Appropriate mimetics of HGF/SF could be identified by screening with an HGF/SF antibody to detect which compounds bind thereto or could be produced by molecular modelling. *See* Morgan et al., "Approaches to the Discovery of the Non-Peptide Ligands for Peptide Receptors and Peptidases," in Annual Reports in Medicinal Chemistry (Academic Press 1989), at pages 243 *et seq*.

The metastasis-inhibiting substance of the present invention also can be an antibody or antibody fragment against HGF/SF or Met which inhibits binding of HGF/SF with Met. An "antibody" in accordance with the present invention includes a whole antibody and parts thereof, either alone or conjugated with other moieties. Antibodies indlude polyclonal antibodies, monoclonal antibodies, and single chain antibodies. Antibody fragments are those that bind HGF/SF or Met, including Fab and F(ab)₂ fragments, *inter alia*. The antibodies of the present invention can be made in animals or by recombinant DNA techniques well-known to the skilled artisan.

In one embodiment of the present invention, applicants produce polyclonal antibodies against synthetic peptides corresponding to Met extracellular domains involved in ligand binding or the modulation of ligand binding. *See* Figures 6 and 7 and description thereof.

Applicants have developed a protocol for the *in vitro* testing of inhibitors of ligand binding directed against Met which measures the ability to block tumor invasion or metastasis. For example, applicants have discovered that NIH/3T3 cells transfected with HGF/SF and *met*^{hu} are "invasive" in a Boyden-chamber assay, as are cells with *met*^{mu} (which produce murine HGF/SF endogenously) and the oncogenic form of *met, tpr-met.* Similarly, *met*^{hu} transfectants are invasive when in "conditioned medium," i.e. medium containing HGF/SF^{hu}. Applicants have further discovered that "1242" antibodies generated against a peptide sequence within the extra-cellular domain of Met, blocks the invasive potential of the tumor cells.

Indeed, chimeric mouse/human experiments, such as those disclosed in Example 2 and discussed with reference to Figure 5, enable the definition of domains of the Met receptor and HGF/SF which are involved in ligand/receptor binding. Through mathematical, computer assisted computations, such as those shown and described under Figure 6, it is possible to define regions within the domains which are different in the mouse and human. These "differences" may modulate species specificity in responses to HGF/SF and antibodies against such regions can be tested for their ability to inhibit HGF/SF binding. Thus, applicants have provided a method for *in vitro* testing of tumor metastasis inhibition applicable to the development of compositions for *in vivo* use.

The delivery of the tumor metastasis-inhibiting substance of the present invention to the selected site of action may be achieved using conventional methods of drug delivery, gene transfer, or any combination thereof. *See* K.J. Van Zee et al. PNAS 89: 4845-49 (1992). Means for delivery include conjugation to a carbohydrate or carrier protein; administration with any slow release complex recognized in the field; compounding in other delivery systems, such as microspheres or liposomes; or adminstering in an expression vector system. One method of delivery applicable to the present invention involves the coupling of the tumor metastasis-inhibiting substance of the present invention to polyethylene glycol or polypropylene glycol to produce a physiologically active non-immunogenic water soluble composition, according to the method of Davis et al., U.S. Patent No. 4,179,337.

Administration of such a composition may be accomplished by any method known to the skilled artisan. For instance, the composition may be in an aqueous solution which is injected into the mammalian circulatory or intramuscular system. The determination of the proper dosage depends upon a number of case specific variables, including the age and weight of the mammal, and involves routine experimentation within the expertise of the skilled artisan.

Artificial activation of the HGF/SF-Met pathway provides the basis for therapeutic methodologies designed to restore, replace, or enhance naturally occurring biological activities. These methodologies include delivery to the site of activation HGF/SF or Met variants or mimetics which enhance the binding interaction between Met and HGF/SF and thereby create an artificially sustained HGF/SF-Met interaction. For example, site-directed mutagenesis of the HGF/SF -binding domain of Met, or the Met-binding domain of HGF/SF (or both) may be used to create a member of the HGF/SF-Met pair with higher binding affinity for the other member of the pair and thus affect accelerated growth or regeneration of wounded tissue. Similarly, conventional recombinant DNA techniques could be used to enhance or sustain the kinase activity of the Met protein normally regulated by HGF/SF binding, including Met mutations possessing a constitutively activated tyrosine kinase. Activation of the HGF/SF-Met pathway by means of supplementing the natural expression of Met by recombinant DNA techniques in combination with exogenously administered HGF/SF is also possible.

Delivery of genetically engineered HGF/SF or Met species to the selected site of action can be achieved using conventional methods of drug delivery, gene transfer, or any combination therof, as discussed above in connection with the delivery of substances which inhibit tumor metastasis.

A method for producing human HGF/SF is presented. Specifically, applicants have discovered that mouse fibroblast NIH/3T3 cells express surprisingly high levels of HGF/SF from a transfected long terminal repeat (LTR) vector recombinant construct containing human HGF cDNA. Applicants have discovered that the highest levels of HGF are detected in NIH/3T3 cells when an LTR human *c-met* proto-oncogene (cDNA vector) is co-transfected with the human LTR/HGF construct and cells are derived from secondary tumors. *See* Example 2, below. One advantage of this cell line is that the transformed cells can grow to high cell density. Accordingly, this cell line produces extremely high levels of HGF/SF, about 1mg of HGF/SF per liter (1250 units per ml). In comparison, another cell line derived from human keratinocytes, ndk cells, produces approximately 10µg per liter per 48 hours. *See* J. C. Adams, et al., Science 98: 385-394 (1991); E.M. Rosen, et al., BBRC 168(3): 1082-1088 (1990). It is therefore unexpected to obtain the yield of 1mg/liter of HGF/SF from NIH/3T3 cells co-transfected with the recombinant vector constructs of the present invention.

A method of producing HFG/SF comprises the steps of:
(a) transfecting NIH/3T3 cells with DNA encoding HGF/SF^{hu} and Met^{hu};
(b) introducing cells transfected in accordance with step (a) into a mammal, thereby generating a primary tumor;
(c) explanting and propagating cells of the primary tumor *in vitro*;
(d) selecting those cells propagated in accordance with step (c) which express high levels of HGF/SF^{hu} and high levels of Met^{hu};
(e) introducing cells selected in accordance with step (d) into a mammal thereby producing a secondary tumor;
(f) explanting and propagating cells of the secondary tumor *in vitro;* and
(g) obtaining HGF/SF^{hu} produced by the cells of step (f).

The DNA comprises a first DNA vector comprising, in operable linkage, a promoter derived from a long terminal repeat of a retrovirus, DNA encoding the entire coding domain of human Met and a polyadenylation signal and a second DNA vector comprising, in operable linkage, a promoter derived from a long terminal repeat of a retrovirus, DNA encoding human HGF/SF and a polyadenylation signal.

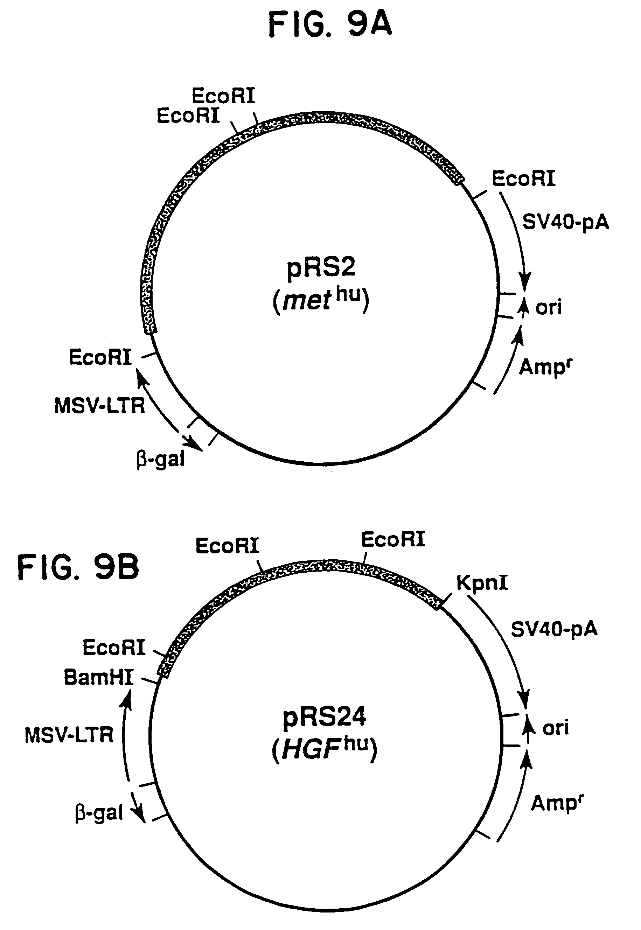

Although the production of the DNA may be accomplished by a variety of methods known to the skilled artisan, production is exemplified in Example 2, in the discussion of cDNA plasmid constructs and cell lines. The preferred DNA vectors of the present invention are depicted in Figure 9.

Cells transfected with HGF/SF^{hu} and Met^{hu} LTR-cDNA are introduced into mammals, according to methods well-known in the art, preferably by injection. *See* Blair, D.G., et al., Science 218: 1122-1125 (1982). The preferred mammals of the present invention are nude mice. Primary tumors which develop in these mammals after 5-10 weeks are explanted and propagated in *in vitro* culture, as described in Example 2, with regard to "nude mouse assays" *In vitro* propagated primary tumor cells are then subjected to immunoprecipitation analysis to ascertain which cells expressed high levels of HGF/SF^{hu} and high levels of Met^{hu}. See Figure 3 and the above description thereof. For instance, explanted cells may be metabolically labeled and then immunopreciptated with Met^{hu} monoclonal antibodies (monoclonal 19S), concentrated, then immunopreciptated with HGF/SF^{hu} monoclonal antibody A3.1.2. High levels of HGF/SF^{hu} and Met^{hu} are determined with reference to expression levels in the starting material. Thus, any level of expression higher than that observed in the starting material is considered "high" for purposes of this method. Cells expressing high levels of HGF/SF^{hu} and Met^{hu} are then introduced into mammals and secondary tumors which develop are explanted and propagated *in vitro*, according to methods well-known in the art. *See* Blair, D.G., et al., Science 218: 1122-1125 (1982).

HGF/SF^{hu} expressed by the explanted secondary tumor cells are then purified as described in Weidner et al., J. of Cell Biol. 111: 2097-2108 (1990), although other methods applicable to the present invention are well-known to the skilled artisan.

Three c-terminal isoforms of mouse and human Met derived from NIH/3T3 cells transfected with met cDNA are disclosed.

Although these isoforms most likely resulted from post-translational processing, applicants cannot exclude possible rearrangements of the transfected cDNA. Several *met* products truncated in the C-terminal have been reported (Prat, M., et al. Mol. Cell. Biol. 11: 5954-5962 (1991)) but these products are different from the isoforms disclosed herein, because they do not react with C-terminal antibodies. Moreover, these isoforms are detected only in cells expressing high levels of *met*, indicating that they are present in low abundance.

### Example 1. The c-met proto-oncogene is the receptor associated with motogenicity.

The ability of HGF/SF to induce mitogenesis and/or scattering (motogenesis) in a number of human carcinoma cell lines that express *c-met* receptor was examined. Cell lines shown in Table 1, above, were plated in 96 well tissue culture plates at 1 X 10⁴- 5 X 10⁴ cells/ml and allowed to grow to near confluency. Wells were washed free of serum and cells starved for two days in serum-free media. The following day, cells were treated with either no sera, sera, or 10ng/ml HGF/SF for 16-18 hours, and then 1µCi of ³H-thymidine was added per well (5µCi/ml) for another six hours. The assay was terminated by washing wells free of label with ice-cold PBS, fixing the cells in 5% TCA, and solubilizing the DNA with 0.25 M NaOH. Samples were then counted in scintillation vials. The mitogenicity index is the ratio between thymidine incorporated in the absence of sera versus that stimulated by HGF. For motogenicity assays, cells were plated at 2 X 10³/ml in 16-well chamber slides (LabTek) and grown until about 60% confluent. Cells were than washed free of serum and incubated overnight in serum-free media +/- HGF/SF (10ng/ml). Slides were fixed with ice cold acetone for 10 minutes, stained with crystal violet (5 minutes), and rinsed well with water. Qualitative changes in "scattered phenotype" were recorded.

To demonstrate that the introduction of *c-met* into an epithelial cell line confers "scattering" activity onto the cells, C127 cells were transfected by lipofection (BRL) with either human *met* cDNA or murine *met* cDNA. Mitogenicity and/or motogenicity was measured as in Table 1. The results of this study are set forth in Table 2, above.

### Example 2. Production of HGF/SF in NIH/3T3 Cell Line

**cDNA plasmid constructs and cell lines**. *Met* cDNA plasmids were constructed in pMB1, a derivative (without the polylinker sequences) of pMEX, Oskam, R., et al. Proc. Nat'l. Acad. Sci., USA 85: 2964-2968 (1988); that contains the long terminal repeat (LTR) promoter from Moloney murine sarcoma virus (MSV) and the polyadenylation signal of simian virus 40. The met^{hu} plasmid was constructed by replacing an internal 300-bp EcoRI fragment with the 250-bp EcoRI fragment of pOK in the 4.6Kb *met*^{hu} sequence containing the open reading frame. Park, M., et al. Proc. Nat'l. Acad. Sci. USA 84: 6379-6383 (1987); Rodriguez et al., Mol. Cell Biol. 11: 2962-2970 (1991). The met^{mu} plasmid contains the entire 4.6-Kb mouse *met* open reading frame. Iyer, A., et al. Cell Growth & Diff. 1:87-95 (1990). Chimeric human/mouse *met* constructs were made using the conserved PvuII site (amino acid 807). A *HGF^{hu}* plasmid was constructed by inserting the 2.3 kb *Bam*H1-*Kpn*I fragments of the human *HGF* sequence into the *Bam*H1-*Kpn*I sites of pMEX. Nakamura, T., et al. Nature 342: 440-443 (1989); Oskam, R., et al. Proc. Nat'l. Acad. Sci., USA 85: 2964-2968 (1988). NIH/3T3 490 cells were grown in DMEM (Gibco) with 8% calf serum (Gibco).

**DNA transfection**. The calcium phosphate method of DNA transfection (Cooper, C.S., et al. Nature 311:29-33 (1984)) was carried out by mixing plasmid DNA (2 µg in 75 µl of water containing 8 µg of calf thymus carrier DNA) with 75 µl of 0.67 M CaCl₂. This mixture was added dropwise to 0.15 ml of solution H (0.27 M NaCl, 0.01 M KCl, 0.0014 M Na₂HP0₄.7H₂0, 0.012 M dextrose) with continuous agitation. After remaining at room temperature for 30 minutes, the mixture was added to cells having about 70% confluence on a 35-mm dish containing 1 ml medium with 0.01 M Hepes buffer. Cells were incubated at 37°C for 4 hours, then treated with 15% glycerol (v/v) in solution H for 2 minutes. For G418 selection, cells were re-fed with DMEM and 8% calf serum overnight and subsequently transferred to three 60mm dishes. After 24 hours incubation, cells were fed with medium containing 400 µg/ml G418 (Gibco) twice a week.

**Northern analysis**. RNA was isolated using RNAsol (CINNA/BIOTECX). Twenty micrograms of RNA was subjected to electrophoresis on 1% denaturing formaldehyde agarose gels followed by transfer to nitrocellulose filters (Schleicher and Schuell). Blots were hybridized at 42°C for 15 hours to ³²P-labeled randomly primed DNA probes (10⁹ cpm/µg) in 30% formamide, 6x saline sodium citrate (SSC), 5x Denhardt's solution, 50mM sodium phosphate (pH 6.8), and sonicated salmon sperm DNA (250 µg/ml). After hybridization, filters were washed twice in 1x SSC and 0.1% SDS at room temperature and in 1x SSC and 0.1% SDS at 50°C. Filters were dried and exposed to X-ray films for 1-3 days at -70°C.

**Immunoprecipitation**. Near-confluent cells were labeled with 0.25 mCi of Translabel (ICN) (1 ml/35-mm dish) for 4 to 6 hours in DMEM lacking methionine and cysteine (Gibco). The labeled cells were lysed in 0.5 ml of RIPA buffer [1% Triton X-100, 1% sodium deoxycholate, 0.1% sodium dodecylsulfate (SDS), 0.15 M NaCl, 0.02 M NaPO₄, pH 7.2, 1 mM phenylmethylsulfonyl fluoride (PMSF), 2 mM EDTA, 50 mM NaF, 30 mM sodium pyrophosphate]. Clarified lysates having equal radioactive counts were immunoprecipitated with 19S monoclonal anti-met antibody, Faletto, D.L. et al. Oncogene 7: 1149-1157 (1991) at 4°C overnight. Immunoprecipitates were complexed to protein G-sepharose (Gibco), then washed twice with RIPA buffer and high-salt buffer (1 M NaCl, 10 mM Tris-HCl, pH 7.2, 0.5% Triton). The immunocomplexes were solubilized by boiling in SDS sample buffer in the presence of 5% β-mercaptoethanol. Samples were analyzed by SDS-polyacrylamide gel electrophoresis followed by treatment with fluorographic enhancer (Amplify^{™}, Amersham) and fluorography with an intensifying screen at -70°C.

SP260 is a peptide antibody made from rabbit antisera, directed against the C-terminal 21 amino acids of *met*^{mu}. (Iyer, A., et al. Cell Growth & Diff. 1: 87-95 (1990)). A3.1.2 is an anti-human recombinant HGF monoclonal antibody (IgG, subclass G2a).

**Pulse chase analysis**. Near-confluent cells were labeled for 45 min with 0.25 mCi of Translabel in 1 ml (per 35-mm dish) DMEM lacking methionine and cysteine. The cells were washed twice and chased with complete medium for 3 hours, then lysed and subjected to immunoprecipitation analysis.

**Surface iodination**. Near-confluent cells were labeled with Na ¹²⁵I in the presence of Iodo-Gen (Pierce). Twenty microliters of the Iodo-Gen reagent (10 mg/ml in chloroform) was added to the bottom of a I-dram vial and dried with a stream of nitrogen. The Iodo-Gen was then dissolved in 1 M Tris with 10 mM EDTA (pH 7.5) and added to the cell pellet. Na ¹²⁵I(0.5 mCi) was added to the reaction for 10 minutes. The labeled cells were washed three times with PBS, then lysed with RIPA buffer and subjected to immunoprecipitation analysis.

**Western analysis**. Near-confluent cells on a 100-mm dish were washed twice with cold TBS (10 mM Tris pH 8.0, 150 mM NaCl) and lysed in 1 ml of lysis buffer (25 mM Tris-HCl, pH 8.0, 100 mM NaCl, 50 mM NaF, 1% Triton X-100, 10 µg/ml aprotinin, 10 µg/ml leupeptin, 1.25 mM PMSF, 1 mM vanadate). One milligram of protein was immunoprecipitated with anti-C28 anti-Met^{hu} polyclonal antibody (Gonzatti-Haces, M. et al., Proc. Nat'l. Acad. Sci. U.S.A., 85: 21-25 (1988)) and subjected to Western analysis with either anti-phosphotyrosine (anti-P-Tyr) antibody, 4G10 (Morrison, D.K., et al., Cell, 58: 649-657(1989)), or human or mouse *met*-specific antibodies, 19S monoclonal (Faletto, D.L., et al., Oncogene 7: 1149-1157 (1991)) or SP260 (Iyer, A., et al., Cell Growth & Diff. 1: 87-95 (1990)), respectively. ¹²⁵I-protein A (Amersham) was used to detect positive bands according to the manufacturer's instructions.

**Nude mouse tumor assay**. The assay was performed as previously described (Blair, D.G., et al., Science 218: 1122-1125 (1982)). Transfected and G418 selected NIH/3T3 cells (10⁶) were washed twice and resuspended in 0.1 ml of serum-free medium. The cells were injected onto the back of weanling athymic nude mice (Harlan Sprague Dawley, Inc.). Tumor formation was monitored each week for up to 10 weeks. Tumors were explanted when they reached 15 mm in size, and the tumor cells were subjected to immunoprecipitation analysis.

**Soft-agar assay.** Soft-agar growth assay was carried out by modification of Blair et al., Virology 95: 303-316 (1979). Briefly, trypsinized cells were suspended at 2 x 10⁵ and 2 x 10⁴ cells in 8 ml of DMEM (Gibco) with 10% calf serum and 0.24% purified agar (DIFCO) and quickly transferred to a duplicate 60-mm dish containing a hardened base layer of DMEM with 10% calf serum and 0.27% agar. Plates were fed with 2 ml of DMEM with 10% calf serum and 0.27% agar at weekly intervals. Colonies were counted microscopically after 3 weeks incubation at 37°C.

**Expression of Met^{hu} and Met^{mu} in NIH/3T3 cells.** Plasmids containing *met*^{hu} protooncogene cDNA were cotransfected with pSV2neo into NIH/3T3 cells and G418 - resistant cells were screened for human or mouse Met expression by immuno-precipitation analyses. These analyses show that both human and mouse p170^{met} and p140^{met} are expressed in transfected NIH/3T3 cells (Fig. 1A, lanes 2 and 3, respectively). Similar levels of *met* expression in NIH/3T3 cells has been reported (Iyer, A., et al., Cell Growth & Diff. 1:87-95 (1990)). Applicants found little or no expression of the endogenous Met^{mu} in the G418-resistant cells expressing Met^{hu}. Appropriate processing of Met^{hu} and Met^{mu} was examined by pulse-chase labeling experiments and these studies showed that p170^{met} synthesized during a 45 minute pulse (Fig. 1B, lanes 1 and 3) was efficiently processed after 3 hours into the mature p140^{met} (lanes 2 and 4). Moreover, human and mouse Met were localized on the cell surface. Applicants labeled intact cells with Na¹²⁵I and immunoprecipitation of the lysates showed that both forms, p140^{met} and p170^{met} were iodinated (Fig. 1C). Thus, human or mouse Met expressed in NIH/3T3 cells is correctly processed and localized on the cell surface. These analyses also show that p170^{met} arrives at the cell surface uncleaved. The iodination of p170^{met} did not result from lysed cells, since under similar iodination conditions the cytoplasmic *tpr-met* oncoprotein was not detected. (Gonzatti-Haces, M., et al., Proc. Nat'l. Acad. Sci. U.S.A. 85: 21-25 (1988)). Furthermore, p170^{met} expressed in Okajima cells, a human gastric carcinoma cell line that overexpresses met^{hu} is also labeled by surface iodination (data not shown), but the ratio of p170^{met} to p140^{met} labeled was less when compared with NIH/3T3 cells.

### Constitutive tyrosine phosphorylation of met in

**NIH/3T3 cells**. By Northern hybridization analyses, applicants detected *HGF*/*SF* mRNA expression in NIH/3T3 cells and in cells transfected with either *met*^{hu} or *met*^{mu}. Applicants observed the same level of HGF/SF mRNA expression, suggesting that overexpression of *met*^{hu} or *met*^{mu} in the G418 selected lines did not affect endogenous *HGF*/*SF* expression. These results also suggested that Met might be activated in an autocrine fashion. Therefore, applicants examined whether the Met^{hu} and Met^{mu} expressed in these cells reacted with anti-P-Tyr antibody. Extracts from cell lines expressing Met^{hu} and Met^{mu} were subjected to immunoprecipitation with human- or mouse-specific peptide antibodies followed by Western analyses using either anti-P-Tyr antibody (Fig. 2A and B), or human (Fig. 2C), or mouse-specific (Fig. 2D) Met antibody. These analyses show that both p170^{met} and p140^{met} of Met^{hu} and Met^{hu} reacted strongly with anti-P-Tyr antibody. This is the first example demonstrating tyrosine phosphorylation of p170^{met} react with anti-P-Tyr antibody. One line expressed Met^{hu} at very high levels (Fig. 2A and C, lane 1). This was an exception since all other Met^{hu} lines expressed levels comparable to lines analyzed in Fig. 2A and C, lanes 2 and 3. Additional C-terminal Met protein products (p85, p75, and p65) were detected with C-terminal antibodies in cells expressing either mouse or human *met* (Fig. 2A-D, lane 1).

**Tumorigenicity of *met* in NIH/3T3 cells**. Applicants observed that NIH/3T3 cell cultures expressing Met^{mu}, but not Met^{hu}, were transformed (Iyer, A., et al., Cell Growth & Diff. 1: 87-95 (1990)) as shown in Figure 5. This was confirmed by testing for their tumorgenicity in nude mice following transfection and G418 selection. NIH/3T3 cells expressing *met*^{mu} were highly tumorigenic as shown in Table 3, below, while cells expressing *met*^{hu} were low in tumorigenicity and only in one line out of eight tested produced tumors.

**Table 3.**

| Tumorigenicity of NIH/3T3 Cells Transfected With *met*^{bu} or *met*^{mu} cDNA | | | |
|---|---|---|---|
| Cells* | Transfected genes | Mice with tumors/ No. tested | Latency (weeks) |
| NIH/3T 3 | neo^{r} | 0/8 | |
| 123-1 | neo^{r}, *met*^{hu} | 2/23 | 5 |
| 121-5 | neo^{r}, *met*^{mu} | 12/12 | 3-5 |

| | | | |
|---|---|---|---|
| Cells (10⁶) were washed twice with serum-free medium and injected subcutaneously on the back of weanling athymic nude mice. Tumor formation was monitored each week up to 10 weeks. | | | |

These tumorigenic lines, before injection, produced high levels of Met^{hu} (Fig. 2A and C, lane 1) compared to lines that were not tumorigenic (Fig. 2A and C, lanes 2 and 3). The tumor explants of this line displayed even higher levels of Met^{hu}. The levels of a Met^{mu} line before injection is shown for comparison (Fig. 2B and D, lane 1). Cell lines expressing lower levels of Met^{mu} are also tumorigenic in this assay. The two tumors generated by line 123-1 (Table 3) showed increased levels of endogenous Met^{mu} expression and reduced levels of Met^{hu}, suggesting that they may have arisen through mouse *met* amplification as previously described. Cooper, C.S., et al., EMBO J. 5: 2623-2628 (1986); Heldin, C.-H. et al., Eur. J. Biochem. 184: 487-496 (1989). Applicants conclude that Met^{hu} is poorly tumorigenic in NIH/3T3 cells.

**Tumorigenicity of NIH/3T3 cells cotransfected with** *met*^{hu} and *HGF*/*SF*^{hu}*.* One explanation for the low tumorigenicity of *met*^{hu}- transfected NIH/3T3 cells is that *met*^{hu} receptor activation by endogenous HGF/SF^{mu} may not provide sufficient signal. Applicants therefore tested whether transfection of both met^{hu} and *HGF*/*SF*^{hu} cDNAS would increase tumorigenicity through an autocrine mechanism. These analyses show that NIH/3T3 cells cotransfected with *met*^{hu} and *HGF*/*SF*^{hu} are highly tumorigenic (Table 4).

**Table 4.**

| Tumorgenicity of NIH/3T3 Cells Transfected With *met^{hu}* ± *HGF*/*SF^{hu}* cDNAs | | | |
|---|---|---|---|
| Cells | Transfected genes | Mice with tumors/No. tested | Latency (weeks) |
| NIH/3T3 | neo^{r} | 0/6 | - |
| 132-4 | neo^{r}, *met*^{hu} | 0/6 | - |
| 132-3, 137-5 | neo^{r}, *met*^{hu}, HGF^{hu} | 17/19 | 4-6 |
| 137-4 | neo^{r}, HGF^{hu} | 3/7 | 7 |

Moreover, the tumor cells showed increased levels of both Met^{hu} and HGF/SF^{hu} (Fig. 3A and C, respectively; lanes 2, 4, and 5). In the tumor explants characterized in lanes 4 and 5, high levels of both Met^{hu} and HGF/SF^{hu} are expressed. None of the cell lines showed amplification of endogenous *met*^{mu} (Fig. 3B). Applicants found that *HGF*/*SF*^{hu}-transfected NIH/3T3 cells also produced several tumors, but the levels of *HGF*/*SF*^{hu} product expressed in tumor cells derived from explants was not as high as the levels expressed in tumors from the *met*^{hu}*-HGF*/*SF*^{hu} cotransfection experiments (Fig. 3C). In one out of five HGF/SF^{hu} tumors, elevated levels of endogenous *met*^{hu} was detected.

**Tumorigenicity of chimeric human/mouse *met* in NIH/3T3 cells**. To test whether the ligand binding domain influenced tumorigenicity, applicants generated chimeric human/mouse *met* receptor molecules and tested their tumorigenicity in nude mice (Figure 5). Applicants used a conserved *Pvu*II site in the external domain adjacent to the transmembrane coding sequences to make these recombinants. It was found that when the mouse external ligand-binding domain was linked to the human transmembrane and tyrosine kinase domains, the chimeric receptor displayed tumorigenic activity equivalent to that of the Met^{mu} (Figure 5). Explants of these tumors showed an increased level of chimeric Met protein that was recognized with human antibody directed against the human tyrosine kinase domain (Fig. 4, lanes 2 and 3). No evidence for *met*^{mu} amplification was observed in these tumors, and the chimeric product was recognized by Western analysis with anti-P-Tyr antibody (Fig. 4, lane 5).

In contrast to the high tumorigenicity of the chimera with the mouse Met external ligand-binding domain, the reciprocal human N-terminal/mouse C-terminal chimera was poorly tumorigenic. However, as with the Met^{hu}, when this chimera was cotransfected with *HGF*/*SF*^{hu} cDNA, efficient tumor formation was observed (Figure 5). Applicants also show that cells expressing the met^{mu}-met^{hu} chimera, before injecting into nude mice, are transformed and form colonies in soft agar like *met*^{mu}-transfected cells. The met^{hu}-met^{mu} cells do not display a transformed phenotype unless coexpressed with HGF/SF. Applicants concluded that the Met^{mu} external ligand-binding domain is a major factor in determining tumorigenicity.

While the foregoing invention has been described in some detail for purposes of clarity and understanding, it will be appreciated by one skilled in the art from a reading of this disclosure that various changes in form and detail can be made without departing from the true scope of the invention.

## Claims

1. The use of a substance which prevents hepatocyte growth factor/scatter factor (HGF/SF) from binding with *met* protooncogene protein (Met), wherein said substance is an antibody or antibody fragment against the extracellular domain of Met, in the preparation of a medicament for use in a method of preventing tumor cell metastasis, wherein in said method, a tumor-bearing mammal is to be treated with an effective inhibiting amount of said substance.

2. The use according to claim 1, wherein said antibody is a polyclonal antibody.

3. The use according to claim 1 or 2, wherein said antibody is against the sequence GKNLNSVSVPRMVINVHEAGRNF.

## Patentansprüche

1. Verwendung einer Substanz, die den Hepatozytenwachstumsfaktor/Scatterfaktor (HGF/SF) hindert, an das *met*-Protoonkogenprotein (Met) zu binden, wobei die Substanz ein Antikörper oder Antikörperfragment gegen die extrazelluläre Domäne von Met ist, zur Herstellung eines Medikaments zur Verwendung in einem Verfahren der Verhinderung von Tumorzellenmetastase, wobei in dem Verfahren ein tumortragender Säuger mit einer wirksamen inhibierenden Menge der Substanz zu behandeln ist.

2. Verwendung nach Anspruch 1, wobei der Antikörper ein polyklonaler Antikörper ist.

3. Verwendung nach Anspruch 1 oder 2, wobei der Antikörper gegen die Sequenz GKNLNSVSVPRMVINVHEAGRNF ist.

## Revendications

1. Utilisation d'une substance qui empêche le facteur de croissance des hépatocytes/facteur de dispersion (HGF/SF) de se lier à la protéine proto-oncogène *met* (Met), dans laquelle ladite substance est un anticorps ou un fragment d'anticorps dirigé contre le domaine extracellulaire de Met, dans la préparation d'un médicament pour une utilisation dans un procédé de prévention des métastases de cellule tumorale, dans laquelle, dans ledit procédé, un mammifère porteur de tumeur doit être traité à l'aide d'une quantité inhibitrice efficace de ladite substance.

2. Utilisation selon la revendication 1, dans laquelle ledit anticorps est un anticorps polyclonal.

3. Utilisation selon la revendication 1 ou 2, dans laquelle ledit anticorps est dirigé contre la séquence GKNLNSVSVPRMVINVHEAGRNF.
